# EUROPÄISCHE PATENTSCHRIFT

(11) **EP 2 719 753 B1**
(45) Veröffentlichungstag und Bekanntmachung des Hinweises auf die Patenterteilung: **25.02.2015**
(21) Anmeldenummer: 12007117.0
(22) Anmeldetag: 11.10.2012
(51) Int. Cl.: C12M 1/00, C12M 1/42

(54) **Reaktor mit Elektrolumineszenzpartikeln im Reaktionsmedium**
Reactor with electroluminescence particles in the reaction medium
Réacteur avec particules à électroluminescence dans un milieu de réaction

(43) Veröffentlichungstag der Anmeldung: 16.04.2014
(73) Patentinhaber: Friedrich-Alexander-Universität Erlangen-Nürnberg, 91054 Erlangen (DE)
(72) Erfinder: Buchholz, Prof. Dr. Rainer, 91093 Hessdorf (DE); Heining, Martin, 91052 Erlangen (DE); Lindenberger, Dr. Christoph, Busan 6178-230 (KR); Manstetten, Dipl. Ing. Paul, 93138 Kareth (DE)
(74) Vertreter: Jungblut, Bernhard Jakob

(56) Entgegenhaltungen:
- EP-A1- 1 088 874
- EP-A2- 0 817 537
- WO-A1-2005/005326
- WO-A1-2008/151376
- WO-A1-2010/129278
- WO-A2-2009/018498
- DE-A1-102009 015 925
- DE-A1-102010 007 168
- DE-C1- 19 747 994

## Beschreibung

### Gebiet der Erfindung

Die Erfindung betrifft eine neue Verwendung von zur Elektrolumineszenz befähigten Partikeln, einen Photoreaktor umfassend einen Reaktionsraum, in welchem ein fluides Reaktionsmedium enthalten ist, Verwendungen eines solchen Photoreaktors, und Verfahren zur Kultivierung von phototrophen Zellen sowie zur Durchführung von Photoreaktionen.

Hintergrund der Erfindung und Stand der Technik.

Photobioreaktoren sind ein besonderes Spezialgebiet im Bereich der Bioverfahrenstechnik, da die Reaktoren hierfür nicht lediglich angepasste Reaktoren auch dem Bereich der chemischen Verfahrenstechnik sind, worin die Optimierung der Reaktionsabläufe lediglich die Transportprozesse und Mischtechniken umfasst. Vielmehr spielen bei Photoreaktoren der Lichteintrag und dessen Verteilung innerhalb des Reaktionsmediums eine erhebliche Rolle, zumal Licht jedenfalls derzeit in wäßrigen Medien nicht dispergierbar ist. Diese technische Herausforderung führte bislang zu diversen Konstruktionen für Photobioreaktoren.

Grundsätzlich kann unterschieden werden zwischen Rührkesselreaktoren (mitunter mit interner Beleuchtung), Röhrenreaktoren, (senkrechte) Säulenreaktoren und Flachplattenreaktoren. Generell erfolgt dabei die Zufuhr von Lichtenergie zum Reaktionsmedium von außen, i.e. durch eine für Licht transparente Reaktorwandung. Reaktoren mit solcher externer Lichtzufuhr lassen sich nur schwer vom Technikumsmaßstab zum Produktionsmaßstab vergrößern, da die Effizienz der Lichtzufuhr und somit der Photobioreaktionen von der Oberfläche der transparenten Reaktorwandung abhängt und somit nicht linear mit einer Vergrößerung des Reaktorvolumens zunimmt. Eine Vergrößerung des Reaktorvolumens führt folglich zu sinkenden Reaktionsraten.

Ein weiteres Problem solcher extern beleuchteter Photobioreaktoren ist, dass die Lichtverhältnisse innerhalb des Reaktionsmediums, welches mit den Zellen Trübstoffe enthält, inhomogen sind und diese Inhomogenität zudem mit wachsender Zelldichte, also Reaktionsfortschritt, noch zunimmt. Daher spielt auch das Durchmischungsverhalten in solchen Photobioreaktoren eine eminent wichtige Rolle. Grundsätzlich wäre es wünschenswert, innerhalb verschiedener Volumeneinheiten des Reaktionsmediums im Wesentlichen gleiche Reaktionsbedingungen zu schaffen.

Schließlich kann die zugeführte Lichtleistung nicht beliebig erhöht werden, da bei vielen phototrophen Mikroorganismen ab einer gewissen Lichtintensität eine Photoinhibierung eintritt. Dies ist folglich der Maximalwert in irgendeinem Volumenelement des Reaktorraumes und in anderen Volumenelementen ist die Lichtleistung absorptionsbedingt dann deutlich niedriger mit der Folge niedrigerer Photobiosyntheseleistung.

Eine aus der Praxis bekannte Bauform ist ein Rührkesselreaktor, in dessen Reaktionsraum Lichtleitfasern hinein führen, welche von außen mit Licht beaufschlagt werden. Das Licht wird durch die Lichtleitfasern in das Reaktionsmedium geführt und dort emittiert. Bei geeigneter Anordnung der Enden der Lichtleitfasern im Reaktionsraum kann eine nahezu homogene Ausleuchtung des Reaktionsmediums erreicht werden. Allerdings behindern die Lichtleitfasern die Massentransportprozesse, Konvektion und Diffusion, aus geometrischen Gründen in erheblichem Maße, wodurch dann wiederum die Homogenität der Verteilung der Reaktanden innerhalb des Reaktionsraumes nachteilig beeinflusst wird und so letztlich die erzielbaren Reaktionsraten. Ein Beispiel eines solchen Reaktortyps ist in der Literaturstelle DE 10 2007 055 569 A1 beschrieben.

Eine weitere Klasse von Photobioreaktoren ist durch die Flachplattenreaktoren gebildet. Lediglich beispielsweise sei auf die Literaturstelle DE 10 2009 015 925 A1 verwiesen. Ein charakteristischer Parameter dieser Konstruktion ist die Dicke der zumindest einseitg transparenten Reaktoren, welche typischerweise im Bereich von 70 mm liegt. Sie werden analog sogenannten "airlift" Reaktoren mit einer Begasung entlang des Reaktorbodens betrieben. Dies ermöglicht ein Durchmischungsverhalten, welches bei optimaler Höhe und Länge des Reaktors bis auf 80% der Mischeffizienz eines idealen Rührkessels herankommt. Dennoch ist der Stofftransport von CO₂ und O₂ nicht sehr effizient aufgrund der kurzen Lebensdauer von Gasblasen. Dies läßt sich durch verschiedene Parameter verbessern, wodurch ein scale-up auf bis zu 133 l möglich wird (siehe die Literaturstelle EP 1 326 959 B1).

Röhrenreaktoren haben grundsätzlich in der Biotechnologie die gleichen Nachteile, wie in der chemischen Prozesstechnik. Der größte Nachteil bei horizontal verlaufenden Röhrenreaktoren ist, dass die Abnahme von Substrat entlang der Fließrichtung des Reaktionsmediums, wodurch folglich die Reaktionsraten abnehmen, zwar für kurze Röhren vernachlässigt werden kann, da dann die Reaktions- bzw. Wachstumskinetik in erster Näherung von nullter Ordnung ist. Dies beschreibt aber nur die Hauptreaktion des Prozesses, beispielsweise die chemischen Eigenschaften von CO₂ in Wasser können den pH Wert erheblich beeinflussen, wodurch das Wachstum gehemmt bzw. verlangsamt werden kann. Auch spielt die erforderliche Entfernung von O₂ aus dem Reaktionsmedium eine das Wachstum beeinflussende Rolle. Alle diese Probleme nehmen bei zunehmender Röhrenlänge stark zu. Dennoch sind solche Photobioreaktoren mit bis zu 500 km Länge und einem photoaktiven Volumen von 600 m³ bekannt. Die Reaktionseffizienz ist jedoch vergleichsweise schlecht aus den genannten Gründen und bei einem up-scale muss die Längenzunahme und somit der technische Aufwand überproportional erhöht werden.

Ein anderer Röhrenreaktortyp ist durch vertikale Röhrenanordnung gebildet, wobei die Röhren U-förmig ausgebildet sind und im Bereich des U-Bogen eine Begasungseinrichtung aufweisen. Hierdurch wird auch der Stofftransport durch die U-Schenkel bewirkt. Für ein Beispiel eines solchen Reaktortyps in der Biotechnologie wird auf die Literaturstelle DE 197 47 994 C1 verwiesen. Zwar ist mit einem solche Reaktortyp ein scale-ip bis zu 120 l möglich, aber auch insofern besteht noch Verbesserungsbedarf.

Hinsichtlich Mischverhalten und Stofftransport wäre ein Rührkesselreaktor ideal, wobei solche Rührkesselreaktoren mit transparenten Wandungen und externer Beleuchtung bislang nur im Labormaßstab als Photobioreaktoren eingesetzt werden und nicht im industriellen Maßstab, aufgrund der Probleme der homogenen Einleitung des Lichts in alle Volumenelemente des Reaktionsraumes. Denn die Dämpfung des Lichtes erfolgt exponentiell mit dem Abstand von der transparenten Reaktorwandung, zumal auch der Extinktionskoeffizient zudem mit zunehmender Zelldichte auch noch zunimmt. Bei einem scale-up wird daher im Kern immer versucht, das Verhältnis von (transparenter) Oberfläche zu Volumen zu erhöhen bzw. zu optimieren. In einer nicht-trüben Flüssigkeit würde die Lichtintensität eines rundum beleuchteten zylindrischen Körpers zu einer erhöhten Intensität in der Mittelachse führen, ähnlich dem Effekt einer Sammellinse. In einer Zellsuspension kann dann eine Zelldichte eingerichtet werden, bei welcher in etwa überall im Querschnitt die gleiche Lichtintensität herrscht. Diese Zelldichte ist jedoch nicht optimal in Hinblick auf die erzielbaren Produktionsleistungen.

Das Dokument WO2010129278 offenbart einen Bioreaktor zu Kultivierung von Zellen, beispielsweise zur Herstellung von Biomasse auf Basis von Algen in einem Photobioreaktor mit Kunstlicht in Form von LED-Arrays in einem Röhrenreaktor. Eine bevorzugte Kunstlichtquelle ist Elektrolumineszenz.. Die LED-Lichtelemente können unterschiedliche Formen im Reaktor ausbilden, beispielsweise zylindrisch. Die Elektrolumineszenzlichtelemente werden zur Emission durch elektromagnetischer Wechselfelder im Reaktionsraum des Reaktors angeregt. Die Elektrolumineszenselemente können in Kegelform, Halbkreises, etc. im Reaktor vorliegen.

Das Dokument WO2005005326 offenbart einen Bioreaktor mit phototrophen Organismen. Es wird ein Nano-Composite-Material offenbart. Die Nano-Composite-Partikel werden einem elektromagnetischen Wechselfeldes im Reaktor ausgesetzt.

Grundsätzlich stellen sich die vorstehenden Probleme nicht nur im Bereich der Photobioreaktoren, sondern auch im Bereich der chemischen Reaktoren für Photoreaktionen chemischer Reaktanden.

### Technisches Problem der Erfindung

Der Erfindung liegt das technische Problem zu Grunde, eine Reaktoranordnung für Photoreaktionen, seien es Photobioreaktionen oder chemische Photoreaktionen, anzugeben, welche eine praktisch homogene Ausleuchtung des Reaktionsraumes und Unabhängigkeit vom Verhältnis der Oberfläche zum Volumen des Reaktionsraums gewährleisten, ohne durch störende Einbauten in den Reaktionsraum den Stofftransport zu behindern oder sonstwie negativ zu beeinflussen.

### Grundzüge der Erfindung und bevorzugte Ausführungsformen

Zur Lösung dieses technischen Problems lehrt die Erfindung die Verwendung von zur Elektrolumineszenz befähigten Partikeln in chemischen, biochemischen und/oder biologischen Reaktoren, wobei der Reaktor zumindest ein Emissionselement zur Emission elektromagnetischer Wechselfelder in einen Reaktionsraum des Reaktors umfasst, wobei die zur Elektrolumineszenz befähigten Partikel in einem in dem Reaktorraum befindlichen flüssigen Reaktionsmedium suspendiert sind, wobei das Reaktionsmedium Photoreaktanden enthält, und wobei die zur Elektrolumineszenz befähigten Partikel durch Energiezufuhr zu dem Emissionselement zur Emission von die Photoreaktanden aktivierendem Licht angeregt werden.

Mit anderen Worten ausgedrückt, die zur Elektrolumineszenz befähigten Partikel sind in dem Reaktionsmedium suspendiert und werden über ein Emissionselement drahtlos zur Elektroluminszenz angeregt. Dadurch, dass die Partikel im Reaktionsmedium verteilt sind und darin zusammen mit den anderen Komponenten des Reaktionsmediums zirkulieren, erfolgt eine nahezu homogene Ausleuchtung des Reaktionsraumes, und zwar ohne dass die Partikel den mit üblichen Maßnahmen für die betreffende Reaktion optimierten Stofftransport innerhalb des Reaktionsraumes negativ beeinflussen. Irgendwelche Verhältnisse Oberfläche zu Volumen des Reaktionsraumes werden irrelevant und ein scale-up mit proportional steigender Produktbildung ist problemlos möglich. Überraschenderweise wurde in bezug auf phototrophe Zellen bzw. Mikroorganismen gefunden, dass elektrische und/oder magnetische Wechselfelder auch hoher Energie in den später erläuterten Frequenzbereichen das Wachstum nicht messbar beeinflussen.

Grundsätzlich kann das Reaktionsmedium flüssig oder im Wesentlichen gasförmig, beispielsweise ein Aerosol, sein Vorzugsweise handelt es sich um eine Flüssigkeit, wäßrig und/oder organisch, wobei für Photobioreaktionen wäßrige Reaktionsmedien zweckmäßig sind.

Die Photoreaktanden können lebende phototrophe Zellen oder Organismen, insbesondere aquatische Mikroorganismen, vorzugsweise Mikroalgen oder Cyanobakterien sein, oder zumindest eine chemische Substanz umfassen, welche sich durch Absorption von Licht in einen angeregten Singulett- oder Triplettzustand bringen läßt und die dabei aufgenommene Energie durch chemische Reaktion mit in dem Reaktionsmedium befindlichen anderen Reaktanden wieder abgibt.

Typische phototrophe Zellen und/oder Mikroorganismen, deren Kultivierung in industriellem Maßstab wünschenswert ist umfassen: Cyanophyta (z.B. Arthrospira platensis, Lyngbya taylorii und Nostoc ellipsosporum), Chlorophyta (z.B. Chlorella vulgaris, Haematococcus pluvialis, Dunaliella salina, Chlamydomonas reinhardtii), Rhodophyta (z.B. Porphyridium purpureum), Heterokontophyta (z.B. Ochromonas danica), Haptophyta (z.B. Isochrysis galbana), Dinophyta (z.B. Amphidinium carterae). Hierbei kann es sich um Zellen bzw. Mikroorganismen handeln, welche selbst das angestrebte Produkt darstellen, aber auch Zellen bzw. Mikroorganismen, welche ein angestrebtes Produkt bilden bzw. exprimieren. In jedem Fall werden dem Reaktionsmedium typischerweise kontinuierlich oder diskontinuierlich Energie liefernde Stoffe zugeführt, deren Zusammensetzung, Art bzw. Mittel der Zugabe und Dosierung nach Maßgabe des betreffenden Zell- bzw. Mikroorganismustyps vom Fachmann unschwer gewählt werden kann. Sofern zu kultiviernde Zellen bzw. Mikroorganismen Stoffe bilden, welche das Zellwachstum oder eine Produktexpression inhibieren oder hemmen, kann es vorgesehen werden, dass solche Stoffe kontinuierlich oder diskontinierlich entfernt werden. Dies kann beispielsweise durch immobilsierte und mit dem Reaktionsmedium kontaktierte Stoff-spezifische Absorptions- oder Reaktionsmitteln oder durch nach Maßgabe der abzutrennenden Stoffe gewählte Permeationsmembranen erfolgen. Sind die Zellen bzw. Mikroorganismen selbst das Produkt, so wird ein Teil der Population im Reaktionsmedium nach fachüblicher Weise kontinuierlich oder diskontinuierlich abgetrennt. Grundsätzlich sind diesbezüglich alle herkömmlichen Technologien der Bioreaktoren einsetzbar.

Typische im Industriemaßstab durchgeführte chemische Photoreaktionen sind beispielsweise: Photokatalytische Wasserspaltung zur Wasserstoffgewinnung, photochemische Wasser- und Abwasseraufbereitung mittels Wasserphotolyse, Photolyse von Wasserstoffperoxid, Photolyse von Ozon, Photokatalyse mit Titandioxid (TiO₂), Photo-Fenton-Prozess. Auch hier kann die Zufuhr von Edukten und die Abfuhr von Produkten in fachüblicher Weise und nach Maßgabe der jeweiligen Reaktion erfolgen.

Als zur Elektroluminszenz befähigte Partikel kommen alle Konstrukte in Frage, welche in dem einzusetzenden Reaktionsmedium chemisch und physikalisch stabil sind. Erfoderlichenfalls können die Partikeln eine für das von den Partikeln emittierte Licht transparente Umhüllung aufweisen, welche gegenüber dem Reaktionsmedium bzw. dessen Komponenten chemisch inert ist.

Eine mögliche Gruppe von zur Elektrolumineszenz befähigten Partikel enthält elektroluminophore Substanzen und weist vorzugsweise eine Kern-Mantel-Struktur auf, wobei die elektroluminophore Substanz in Kern angeordnet ist und der Mantel aus einem transparenten und im Reaktionsmedium unter Reaktionsbedingungen inerten Werkstoff besteht. Als elektroluminophore Substanzen kommen alle Elektrolumineszenz zeigenden Substanzen in Frage. Hierzu gehören fluoreszierende (Lebendauer des angeregten Zustandes meist kleiner als 10⁻⁶ oder 10⁻⁹ s) oder phosphoreszierende (Lebendauer des angeregten Zustandes meist größer als 10⁻⁶ s) Substanzen. Luminophore können auf anorganischen Systemen, wie Y₂O₂S:Eu oder ZnSr:Cu, aber auch auf organischen Systemen, wie Fluorescein, basieren. Grundsätzlich sind alle bekannten Elektroluminophore geeignet. Eine Auswahl findet sich in der Literaturstelle Ullmann's chemische Enzyklopädie, Wiley Verlag, elektronische Ausgabe, 2004, Stichwort: Luminescent Materials". Bei Elektroluminophoren handelt es sich typischerweise um partikuläre Materialien, welche anorganische Verbindungen der Gruppen II und VI des Periodensystems, beispielsweise ZnS oder CdS, die mit Metallen, wie Cu, Mn, oder Ag dotiert oder aktiviert sind. Ebenso können partikuläre lumineszierende Substanzen auf der Basis von überwiegend mit Mn, Sr oder mit seltenen Erden aktivierten Silikaten, Aluminaten, Phosphaten, Wolframaten, Germanaten, Boraten, etc., insbesondere Substanzen auf der Basis von Zr₂SiO₄:Mn oder auch partikuläre organische Polymere oder Gemische aus den vorgenannten Verbindungen eingesetzt werden. Ergänzend wird auf die Literaturstelle S. Shionoya et al., Phosphor Handbook, insbesondere Kapitel 9, Electroluminescent materials, CRC Press, 1999, verwiesen. Elektrolumineszierende Substanzen emittieren nach Anregung in einem elektrischen Wechselfeld eine sichtbare Strahlung. Wenn eine Lumineszenzsubstanz Elektrolumineszenz zeigt, erfolgt die Emission sichtbaren Lichts vorzugsweise allein oder überwiegend durch die Anregung in einem elektrischen Wechselfeld. Die Partikel der Lumineszenzsubstanz liegen vorzugsweise in Form von mikroverkapselten Verbindungen bzw. Mantel/Kern-Partikel vor, wobei der Kern durch die Lumineszenzsubstanz gebildet ist. Als Materialien für den Mantel kommen sowohl organische Polymere als auch verschiedene Metalloxide in Frage. Die wesentliche Funktion des Mantels besteht in dem Schutz des Kerns vor Umgebungseinflüssen, die der Beständigkeit und Emissionsfähigkeit des Kerns abträglich sein können. Zudem kann mittels des Mantels die Alterungsbeständigkeit erhöht werden. Schließlich kann mittels des Mantels eine Filterfunktion ausgeübt werden, und zwar sowohl bezüglich einfallender Strahlung als auch emittierter Strahlung. So kann beispielsweise im Falle eine elektrolumineszierenden Kerns der Mantel als UV-Filter funktionieren, der eine Lumineszenz bei UV-Einstrahlung zuverlässig unterbindet. Es ist aber auch möglich, eine solche UV-Filterfunktion mittels einer auf den Mantel aufgebrachten Filterschicht zu erreichen. Die Partikelgröße partikulärer Lumineszenzsubstanzen kann grundsätzlich nach Maßgabe des Reaktionsmediums und sonstiger Komponenten darin oder im Reaktionsraum gewählt sein. Die Partikelgröße kann im Bereich von 0,2 bis zu 100 µm, sogar bis zu 500 µm und mehr, betragen.

Die Anrgeung solcher elektrolumineszierender Partikel erfolgt typischerweise in einem elektrischen Wechselfeld, Hierzu können beispielsweise an der Innenwandung des Reaktors Elektroden vorgesehen und verteilt sein, welche mit dem elektrischen Wechselfeld beaufschlagt werden. Hierzu sind dann entsprechende Generatoren vorzusehen, welche das für die betreffenden Partikel in Frequenz und Intensität geeignete Signale erzeugen, mit welchen die mit dem Generator elektrisch verbundenen Elektroden dann beaufschlagt werden.

Die zur Elektrolumineszenz befähigten Partikel können alternativ zumindest ein LED Bauelement sowie zumindest ein elektrisch mit dem LED Bauelement verbundenes Antennenelement zur Aufnahme eingestrahlter elektromagnetischer Wechselfelder und Umwandlung der aufgenommenen Wechselfelder in elektrische Energie aufweisen. LED Bauelemente sind handelsübliche elektronische Bauelemente und sind mit verschiedenen Emissionsfarben erhältlich. Die für eine bestimmte Photobioreaktion oder chemische Photoreaktion geeignete Farbe kann vom Fachmann unschwer ausgewählt werden, sie ergibt sich aus der gewünschten Photoreaktion. Ebenso sind geeignete Antennenelemente, beispielsweise Spulen, handelsüblich oder aus Draht oder als gedruckter oder integrierter Schaltkreis erstellbar. Die Dimensionierung des Antennenelements erfolgt zweckmäßigerweise nach Maßgabe der Frequenz des einzustrahlenden elektrischen Wechselfeldes und der von dem LED Bauelement benötigten elektrischen Leistung. Es ist auch möglich, zwischen dem Antennenelement und dem LED Bauelement einen Speicher für elektrische Energie und ggf. einer elektronischen Schaltung zur konstanten Abgabe der elektrischen Energie aus dem Speicher an das LED Bauelement zu schalten. Hierdurch wird die Lichtemission konstant gehalten bei schwankender Aufnahme elektrischer Energie durch das Antennenelement. Solche Partikel weisen zweckmäßigerweise eine Umhüllung bzw. ein Gehäuse aus einem transparenten und im Reaktionsmedium unter Reaktionsbedingungen inerten Werkstoff. Dieser Werkstoff sollte natürlich permeabel für elektrische Wechselfelder sein.

Das Emissionselement zur Emission elektromagnetischer Wechselfelder kann im Grunde beliebig, also als induktiver und/oder kapazitativer Emitter ausgebildet sein. Es kann außerhalb einer Wandung des Reaktors, vorzugsweise außen an der Wandung anliegend, innen an der Reaktorwandung anliegend oder von der Reaktorwandung beabstandet im Reaktionsraum angeordnet sein. Im ersteren Fall sollte die Reaktorwandung durchlässig für das elektrische und/oder magnetische Wechselfeld bzw. diesen nicht dämpfend und folglich aus einem entsprechenden Werkstoff gebildet sein. Als Reaktorwandungen kommen typischerweise Glaswerkstoffe und organische Polymerwerkstoffe in Frage, welche fachüblich ausgewählt werden. Wenn eine Spule als induktives Emissionselement innenseitig in Verbindung mit einem metallischen Werkstoff der Reaktorwandung vorgesehen ist, kann es sich empfehlen, den Reaktorraum bzw. die Reaktorwandung innenseitig mit einem Werkstoff mit hoher magnetischer Permeabilität auszukleiden, beispielsweise Mu-Metall, damit in der metallischen Reaktorwandung keine störenden Wirbelströme induziert werden.Bei Anordnung der Spule außenseitig der Reaktorwandung kann es sich empfehlen, wiederum außenseitig der Spule eine die Spule außenseitig abdeckende Abschirmung aus einem Werkstoff hoher magnetischer Permeabilität, beispielsweise Mu-Metall, anzuordnen.

Bezüglich beispielsweise der konkreten Dimensionierung von Komponenten der Erfindung, insbesondere Emissionselement und Antennenelement in der Ausführungsform mit LED Bauelement wird auf die Literaturstelle J. Kuipers et al., Sensors and Actuators A 178 (2012) 217-222, verwiesen. Es wird sich auch im Falle von Spulen als Antennenelemente in der Ausfrührungsform mit LED Bauelement empfehlen, dass die Spule aus Teilspulen gebildet wird, deren Spulenachsen in verschiedene Raumrichtungen, insbesondere 3 Raumrichtungen, weisen, da damit optimale Energieaufnahme unabhängig von der Orientierung des Partikels in dem Reaktionsmedium gewährleistet wird.

Als Anregungsfrequenzen der elektrischen und/oder induktiven Wechselfelder kommt ein Bereich von 1 kHz bis 1 MHz, insbesondere 20 kHz bis 500 kHz, beispielsweise 100 kHz bis 200 kHz in Frage. Das Wechselfeld kann dabei auch gepulst ("an"/"aus") werden, mit Pulsfrequenzen im Bereich von 10⁻⁵ Hz bis 1 kHz. Das Tatsverhältnis ("an" zu "aus") dieser Pulsung kann im Bereich von 1:20 bis 10:1 liegen.

Besonders vorteilhaft, aber nicht zwingend, ist es, wenn die Partikel mit einer mittleren Dichte ausgebildet sind, die sich um weniger als 10%, vorzugsweise weniger als 5%, insbesondere weniger als 2%, von der Dichte des Reaktionsmediums unter Reaktionsbedingungen unterscheidet. Dann sind die Partikel dazu befähigt in dem Reaktionsmedium gleichsam zu schweben und es wird aufgrund der sich einstellenden einigermaßen homogenen Verteilung der Partikel auch eine homogene Lichtemission in dem Reaktionsmedium erzielt. Ist die mittlere Dichte der Partikel jedoch deutlich unterschiedlich zu jener des Reaktionsmediums, dann kann dennoch auch eine einigermaßen homogene Verteilung der Partikel, wenn auch nicht ganz so optimal, durch Agitation des Reaktionsmediums erreicht werden.

Die Erfindung betrifft in einem weiteren Aspekt auch einen Photoreaktor umfassend einen Reaktionsraum, in welchem ein fluides Reaktionsmedium enthalten ist, wobei das Reaktionsmedium zur Elektrolumineszenz befähigte Partikel und Photoreaktanden enthält, wobei der Reaktor zumindest ein Emissionelement zur Emission elektromagnetischer Wechselfelder in den Reaktionsraum des Reaktors aufweist, welches an eine Energiequelle angeschlossen ist und wobei die zur Elektrolumineszenz befähigten Partikel durch Energiezuführ zum dem Emissionselement zur Emission von die Photoreaktanden aktivierendem Licht anregbar sind.

Ein weiterer Aspekt der Erfindung betrifft die Verwendung eines erfindungsgemäßen Photoreaktors zur Durchführung von Photoreaktionen, insbesondere zur Kultivierung von phototrophe Zellen oder Organismen, insbesondere aquatische Mikroorganismen, vorzugsweise Mikroalgen oder Cyanobakterien, oder zur Durchführung chemischer Photoreaktionen, wobei die Photoreaktanden zumindest eine chemische Substanz umfassen, welche sich durch Absorption von Licht in einen angeregten Singulett- oder Triplettzustand bringen läßt und die dabei aufnommene Energie durch chemische Reaktion mit in dem Reaktionsmedium befindlichen anderen Reaktanden wieder abgibt.

Ein wiederum weiterer Aspekt der Erfindung ist ein Verfahren zur Kultivierung von phototrophen Zellen oder Organismen, insbesondere aquatische Mikroorganismen, vorzugsweise Mikroalgen oder Cyanobakterien, wobei diese Zellen oder Organismen in einem Reaktionsmedium eines erfindungsgemäßen Photoreaktors unter Energiezufuhr zu dem Emissionselement für eine vorgebene Zeit und unter vorgegebenen Reaktionsbedingungen kultiviert, insbesondere zirkuliert, werden.

Ein weiterer Aspekt der Erfindung lehrt ein Verfahren zur Durchführung chemischer Photoreaktionen, wobei die Photoreaktanden zumindest eine chemische Substanz umfassen, welche sich durch Absorption von Licht in einen angeregten Singulett- oder Triplettzustand bringen läßt und die dabei aufnommene Energie durch chemische Reaktion mit in dem Reaktionsmedium befindlichen anderen Reaktanden wieder abgibt, wobei diese Reaktanden in einem Reaktionsmedium eines erfindungsgemäßen Photoreaktors unter Energiezufuhr zu dem Emissionselement für eine vorgebene Zeit und unter vorgegebenen Reaktionsbedingungen reagiert werden.

Die zum ersten Aspekt der Erfindung angebrachten Erläuterungen gelten analog auch für die weiteren Aspekte der Erfindung. Die Lehre der Erfindung ist grundsätzlich bei allen Reaktortypen einsetzbar, bei Rührkesselreaktoren, Flachplattenreaktoren und Röhrenreaktoren.

Im Folgenden wird die Erfindung anhand von lediglich Ausführungsbeispiele darstellenden Figuren näher erläutert. Es zeigen:
- Figur 1:: Eine schematische Darstellung eines Flachplattenreaktors mit eine elektroluminophoren Substanzen enthaltenden Partikeln und kapazitiver Anregung,
- Figur 2:: Eine schematische Darstellung eines Rührkesselreaktors mit Partikel mit LED Bauelementen und induktiver Anregung,
- Figur 3:: Eine schematische Darstellung eines Röhrenreaktors, mit Spule außensseitig der Reaktorwandung (3a) oder innenseitig der Reaktorwandung (3b)
- Figur 4:: Schematischer Schaltkreis eines Partikels mit LED,
- Figur 5:: Schematische Darstellung des Gesamtaufbaus eines Partikels mit LED, und
- Figur 6:: Schematische Darstellung eines Generatorschaltkreises für induktive und/oder kapazitive Anregung.

### Beispiel 1: Herstellung von elektrolumineszierenden Mantel/kern-Partikeln

Zunächst wird eine Zubereitung mit den folgenden Komponenten hergestellt: 5-70 Gew.-% Harz-/Bindersystem, 5-70 Gew.-% Lösungsmittel, 0-15 Gew.-%, insbesondere 0,1-15 Gew.-% Katalysatoren/Initiatoren, 0-20 Gew.-%, insbesondere 0,1-20 Gew.-% Additive, 0,1 bis 20 Gew.-% Elektrolumineszenzsubstanz oder eine Mischung verschiedener Elektrolumineszenzsubstanzen, wobei die Summe aller Komponenten stets 100 Gew.-% ergibt.

Als Harz-/Bindersystem sind beispielsweise reaktive Monomere, Oligomere, Präpolymere, wie mono-, di-, und/oder trifunktionale Acrylate geeignet. Käufliche Systeme umfassen beispielsweise die Laromer^{®} Serie(BASF), SR-9003 oder SR-415 (Sartomer), Melamintränkharze, wie die Kauramin^{®} oder Kaurit^{®} Serie (BASF), beispielsweise -752, -753, -786 oder -787, oder Polymerdispersionen, wie die Kauro-pal^{®} Serie (BASF), beispielsweise -937 oder 938. Ebenso sind Firnisse auf Basis von Nitrocellulose oder Leinöl einsetzbar.

Als Lösemittel kommen alle in der organischen Chemie üblichen Lösemittel in Frage mit der Maßgabe, dass eingesetzte Pigmente davon nicht auf- oder angelöst werden. Zu nennen wären Alkohole, wie Methanol, Ethanol oder Isopropanol, Ketone, wie Aceton oder 2-Bunanon, Ester, wie Ethylacetat, halogenierte Lösungsmittel, wie Dichlormethan, und/oder Aromaten, wie Toluol oder Xylol. Bei einer Wasser-basierten Zubereitung ist das wesentliche Lösemittel Wasser, wobei in geringen Mengen, typischerweise unter 20-Gew.-%, meist unter 10 Gew.-%, bezogen auf das Lösemittel, auch organische Lösemittel zugegen sein können.

Als Katalysatoren/Initiatoren können übliche Photoinitiatoren, wie Irgacure^{®} 2020, Irgacure^{®} 819 oder Darocure^{®} 1173 (alle Ciba) eingesetzt werden. Für die genannten Kauramin^{®} oder Kauramit^{®} Harze sind die beispielsweise die Härter 527 oder 529 (BASF) geeignet. Auch Radikalbildner, wie beispielsweise Azo-Iso-Butyrodinitril, sind einsetzbar.

Als Additive kommen in Frage Hilfsstoffe, wie Antischaummittel (beispielsweise Byk-020 oder -052 von Byk), Surfactants (beispielsweise Baysilone von Bayer oder Byk-306 oder 310 von Byk), Konservierungsmittel (beispielsweise Borchers S1 von Borchers), etc. Bezüglich geeigneter Additive wird ergänzend auf Ullmann's chemische Enzyklopädie, Wiley Verlag, elektronische Ausgabe 2007, Stichwort "Paint Additives" oder www.borchers.de verwiesen.

Geeignete Elektroluminophore sind beispielsweise in der Literaturstelle S. Shionoya et al., Phosphor Handbook, Kapitel 9, Electroluminescent Materials, CRC Press, 1999, beschrieben. Dabei weist die Lumineszenzsubstanz generell vorzugsweise eine grobkörnige Struktur auf, beispielsweise einen d90-Wert (Durchmesserbereich, welchen 90 Gew.-% der Partikel aufweisen, Rest typischerweise Feinanteil) von 50-500 µm. Idealerweise ist die Größenverteilung sehr eng, insbesondere praktisch monomodal.

Die vorstehend beschriebene Zubereitung wird in Tropfenform ausgehärtet, wodurch Mantel/Kern Partikel 7 erhalten werden.

Man erhält Partikel, welche unter Anregung mit elektrischen Wechselfeldern Lumineszenz zeigen.

### Beispiel 2: Erfindungsgemäßer Flachplattenreaktor

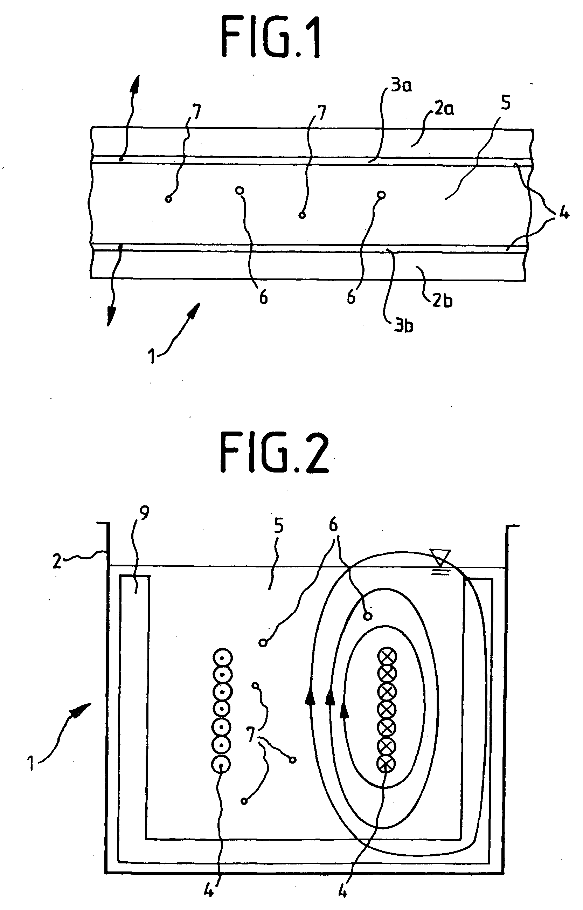

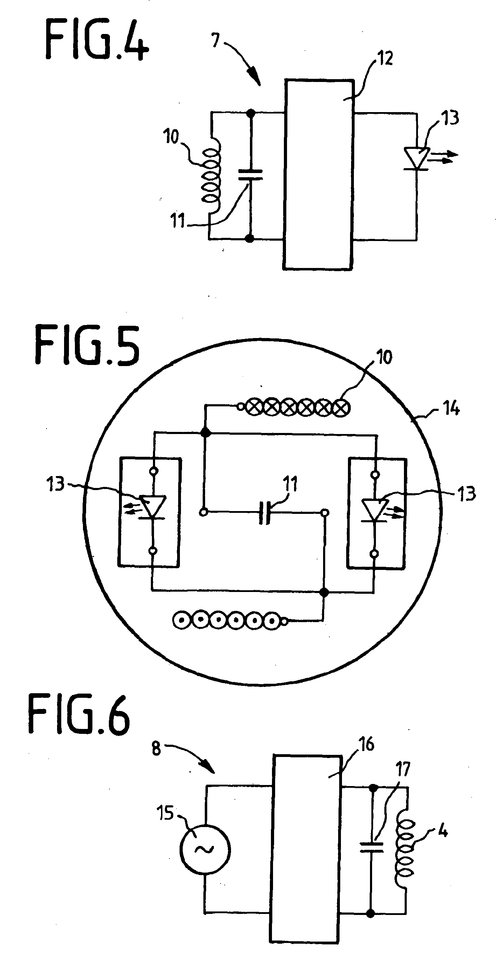

In der Figur 1 erkennt man einen Flachplattenreaktor 1 im Querschnitt senkrecht zu den Hauptflächen. Man erkennt zwei einander in einem Abstand von 70 mm gegenüberliegenden Reaktorwandungen 2a,b. Innenseitig der Reaktorwandungen 2a,b sind zwei Flächenelektroden 3a,b aus einem elektrisch leitfähigen Material angebracht, welche durch eine geeignete nicht-leitende Beschichtung gegenüber dem Reaktionsmedium elektrisch isoliert sind. Die beiden Flächenelektroden 3a,b bilden einen Kondensator 4 als Emissionselement 4 eines Generatorschaltkreises 8, wie in der Figur 6 dargestellt. Zwischen den Reaktorwandungen 2a,b ist der Reaktionsraum 5 angeordnet. Dieser ist mit einem Reaktionsmedium gefüllt, in welchem sich einerseits Mikroorganismen 6 als Photoreaktanden 6 und andererseits die Partikel 7 aus Beispiel 1 suspendiert befinden.Mittel zum Transport des Reaktionsmediums bzw. dessen Durchmischung sind der Übersichtlichkeit halber nicht dargestellt.

Bei Aktivierung des Generatorschaltkreises 8 werden elektrische Wechselfelder von den Flächenelektroden 3a,b in den Reaktionsraum 5 emittiert, welche die darin schwebenden Partikeln 7 zur Luminszenz anregen.

### Beispiel 3: Erfindungsgemäßer Rührkesselreaktor

In der Figur 2 erkennt man einen Rührkesselreaktor 1 im Querschnitt. Man erkennt die den Kessel bildende Reaktorwandung 2. Innenseitig der Reaktorwandung 2 ist eine Spule 4 als Emissionselement 4 eines Generatorschaltkreises 8, wie in der Figur 6 dargestellt, angeordnet. Innerhalb der Reaktorwandung 2 ist der Reaktionsraum 5 angeordnet. Dieser ist mit einem Reaktionsmedium gefüllt, in welchem sich einerseits Mikroorganismen 6 als Photoreaktanden 6 und andererseits LED-Partikel 7 aus Figur 5 suspendiert befinden. Mittel zum Transport des Reaktionsmediums bzw. dessen Durchmischung sind auch hier der Übersichtlichkeit halber nicht dargestellt. Außerhalb oder innerhalb der Reaktorwandung 2 kann eine Abschirmung 9 sein.

Bei Aktivierung des Generatorschaltkreises 8 werden elektrische Wechselfelder von der Spule 4 in den Reaktionsraum 5 emittiert, welche die darin schwebenden Partikel 7 zur Lumineszenz anregen.

### Beispiel 4: Erfindungsgemäßer Röhrenreaktor

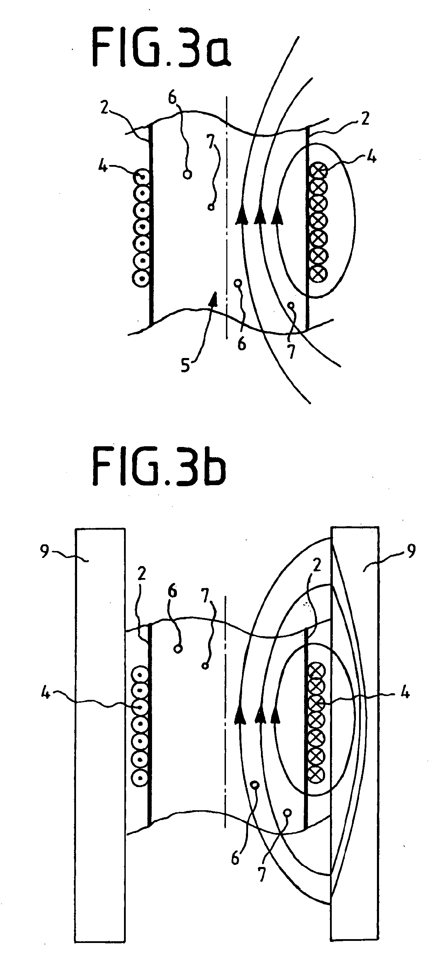

In der Figur 3a erkennt man einen Röhrenreaktor 1 im Querschnitt. Man erkennt die eine Röhre bildende Reaktorwandung 2. Außenseitig der Reaktorwandung 2 ist eine Spule 4 als Emissionselement 4 eines Generatorschaltkreises 8, wie in der Figur 6 dargestellt, angeordnet. Innerhalb der Reaktorwandung 2 ist der Reaktionsraum 5 angeordnet. Dieser ist mit einem Reaktionsmedium gefüllt, in welchem sich einerseits Mikroorganismen 6 als Photoreaktanden 6 und andererseits LED-Partikel 7 aus Figur 5 suspendiert befinden. Mittel zum Transport des Reaktionsmediums bzw. dessen Durchmischung sind auch hier der Übersichtlichkeit halber nicht dargestellt.

Bei Aktivierung des Generatorschaltkreises 8 werden elektrische Wechselfelder von der Spule 4 in den Reaktionsraum 5 emittiert, welche die darin schwebenden Partikel 7 zur Lumineszenz anregen.

In der Figur 3b ist außerhalb der Spule 4, diese umfassend, eine Abschirmung 9 aus einem magnetisch hoch permeablen Werkstoff, wie Mu-Metal, angeordnet. Aus einer vergleichenden Betrachtung der Figuren 3a und 3b erkennt man den Einfluss der Abschirmung 9 auf die magnetischen Feldlinien.

### Beispiel 5: LED-Partikel

In der Figur 4 erkennt man einen schematischen Schaltkreis eines LED-Partikels 7. Dieser umfasst eine als Antennenelement 10 wirkende Spule 10, und einen Kondensator 11, welche einen Resonanzkreis bilden. Hieran angeschlossen ist eine Energiespeicher- und Ladekontrolleinheit 12. An diese wiederum ist die LED 13 angeschlossen.

In der Figur 5 erkannt man wiederum eine Spule 10, einen Kondensator 11 sowie in diesem Beispiel 2 LEDs 13. Selbstverständlich kann auch hier eine Energiespeicher- und Ladekontrolleinheit 12 vorgesehen sein. Die Schaltung ist mit einem Mantel 14 aus einem in dem Reaktionsmedium inerten Kunststoff umgeben bzw. hierein eingegossen.

### Beispiel 6: Generatorschaltkreis

In der Figur 6 erkennt man einen Generatorschaltkreis 8 mit einem Oszillator 15, einem Verstärker 16, einer als Emmissionselement 4 wirkenden Spule 4 und einen Kondensator 17. Alternativ zur Darstellung der Figur 6 können Spule 4 und Kondensator 17 auch in Serie geschaltet sein.

## Patentansprüche

1. Verwendung von zur Elektrolumineszenz befähigten Partikeln (7) in chemischen, biochemischen und/oder biologischen Reaktoren (1),
wobei der Reaktor (1) zumindest ein Emissionselement (4) zur Emission elektromagnetischer Wechselfelder in einen Reaktionsraum (5) des Reaktors (1) umfasst,
wobei die zur Elektrolumineszenz befähigten Partikel (7) in einem in dem Reaktionsraum (5) befindlichen flüssigen Reaktionsmedium suspendiert sind,
wobei das Reaktionsmedium Photoreaktanden (6) enthält, und
wobei die zur Elektrolumineszenz befähigten Partikel (7) durch Energiezufuhr zu dem Emissionselement (4) zur Emission von die Photoreaktanden (6) aktivierendem Licht angeregt werden.

2. Verwendung nach Anspruch 1, wobei das Reaktionsmedium wäßrig und/oder organisch, ist.

3. Verwendung nach Anspruch 1 oder 2,
wobei die Photoreaktanden (6) lebende phototrophe Zellen oder Organismen, insbesondere aquatische Mikroorganismen, vorzugsweise Mikroalgen oder Cyanobakterien, oder
wobei die Photoreaktanden (6) zumindest eine chemische Substanz umfassen, welche sich durch Absorption von Licht in einen angeregten Singulett- oder Triplettzustand bringen läßt und die dabei aufgenommene Energie durch chemische Reaktion mit in dem Reaktionsmedium befindlichen anderen Reaktanden wieder abgibt.

4. Verwendung nach einem der Ansprüche 1 bis 3, wobei die zur Elektrolumineszenz befähigten Partikel (7) elektroluminophore Substanzen enthalten und vorzugsweise eine Kern-Mantel-Struktur aufweisen, wobei die elektroluminophore Substanz in Kern angeordnet ist und der Mantel aus einem transparenten und im Reaktionsmedium unter Reaktionsbedingungen inerten Werkstoff besteht.

5. Verwendung nach einem der Ansprüche 1 bis 3, wobei die zur Elektrolumineszenz befähigten Partikel (7) zumindest ein LED Bauelement (13) sowie zumindest ein elektrisch mit dem LED Bauelement (13) verbundenes Antennenelement (10) zur Aufnahme eingestrahlter elektromagnetischer Wechselfelder und Umwandlung der aufgenommenen Wechselfelder in elektrische Energie aufweisen.

6. Verwendung nach Anspruch 5, wobei die Partikel (7) eine Umhüllung (14) aus einem transparenten und im Reaktionsmedium unter Reaktionsbedingungen inerten Werkstoff aufweisen.

7. Verwendung nach einem der Ansprüche 1 bis 6, wobei das Emissionelement (4) zur Emission elektromagnetischer Wechselfelder als induktiver und/oder kapazitativer Emitter (4) ausgebildet ist.

8. Verwendung nach einem der Ansprüche 1 bis 7, wobei die Partikel (7) mit einer mittleren Dichte ausgebildet sind, die sich um weniger als 10%, vorzugsweise weniger als 5%, insbesondere weniger als 2%, von der mittleren Dichte des Reaktionsmediums unter Reaktionsbedingungen unterscheidet.

9. Photoreaktor (1) umfassend einen Reaktionsraum (5), in welchem ein flüssiges Reaktionsmedium enthalten ist,
wobei das Reaktionsmedium zur Elektrolumineszenz befähigte Partikel (7), welche in dem Reaktionsmedium suspendiert sind, und Photoreaktanden (6) enthält,
wobei der Reaktor (1) zumindest ein Emissionelement (4) zur Emission elektromagnetischer Wechselfelder in den Reaktionsraum (5) des Reaktors (1) aufweist, welches an eine Energiequelle angeschlossen ist und
wobei die zur Elektrolumineszenz befähigten Partikel (7) durch Energiezuführ zum dem Emissionselement (4) zur Emission von die Photoreaktanden (6) aktivierendem Licht anregbar sind.

10. Verfahren zur Kultivierung von phototrophen Zellen oder Organismen (6), insbesondere aquatische Mikroorganismen, vorzugsweise Mikroalgen oder Cyanobakterien, wobei diese Zellen oder Organismen (6) in einem Reaktionsmedium eines Photoreaktors (1) nach Anspruch 9 unter Energiezufuhr zu dem Emissionselement (4) für eine vorgebene Zeit und unter vorgegebenen Reaktionsbedingungen kultiviert, insbesondere zirkuliert, werden.

## Claims

1. Use of particles (7) capable of electroluminescence in chemical, biochemical, and/or biological reactors (1),
wherein the reactor (1) comprises at least one emission element (4) for emitting electromagnetic alternating fields in a reaction chamber (5) of the reactor (1),
wherein the particles (7) capable of electroluminescence are suspended in a liquid reaction medium located in the reaction chamber (5),
wherein the reaction medium contains photoreactants (6), and
wherein the particles (7) capable of electroluminescence are excited by means of supply of energy to the emission element (4) to emit light that activates the photoreactants (6).

2. The use according to claim 1, wherein the reaction medium is aqueous and/or organic.

3. The use according to claim 1 or 2,
wherein the photoreactants (6) comprise living phototrophic cells or organisms, in particular aquatic microorganisms, preferably micro-algae or cyanobacteria, or
wherein the photoreactants (6) comprise at least one chemical substance, which can be excited by absorption of light into an excited singlet or triplet state and releases the received energy by a chemical reaction with other reactants in the reaction medium.

4. The use according to one of claims 1 to 3, wherein the particles (7) capable of electroluminescence contain electroluminophorous substances and preferably have a core-sheath structure, wherein the electroluminophorous substance is located in the core, and the sheath consists of a transparent material under reaction conditions being inert in the reaction medium.

5. The use according to one of claims 1 to 3, wherein the particles (7) capable of electroluminescence comprise at least one LED component (13) and at least one antenna element (10) electrically connected with the LED component (13) for receiving irradiated electromagnetic alternating fields and converting the received alternating fields into electric energy.

6. The use according to claim 5, wherein the particles (7) comprise an enclosure (14) of a transparent material under reaction conditions being inert in the reaction medium.

7. The use according to one of claims 1 to 6, wherein the emission element (4) is configured to emit electromagnetic alternating fields as an inductive and/or capacitative emitter (4).

8. The use according to one of claims 1 to 7, wherein the particles (7) are formed with an average density that differs by less than 10 %, preferably less than 5 %, in particular less than 2 %, from the average density of the reaction medium under reaction conditions.

9. A photoreactor (1) comprising a reaction chamber (5), in which a liquid reaction medium is contained,
wherein the reaction medium includes particles (7) capable of electroluminescence, which are suspended in the reaction medium, and photoreactants (6),
wherein the reactor (1) comprises at least one emission element (4) to emit electromagnetic alternating fields in the reaction chamber (5) of the reactor (1), which is connected to an energy source, and
wherein the particles (7) capable of electroluminescence can be excited by means of supply of energy to the emission element (4) to emit light that activates the photoreactants (6).

10. A method for cultivating phototrophous cells or organisms (6), in particular aquatic microorganisms, preferably micro-algae or cyanobacteria, wherein these cells or organisms (6) are cultivated, in particular circulated, in a reaction medium of a photoreactor (1) according to claim 9 under supply of energy to the emission element (4) for a given time and under given reaction conditions.

## Revendications

1. Utilisation de particules (7) capables d'électro-luminescence dans des réacteurs chimiques, biochimiques et/ou biologiques (1),
dans laquelle le réacteur (1) comprend au moins un élément d'émission (4) pour émettre des champs électromagnétiques alternatifs dans une chambre de réaction (5) du réacteur (1),
dans laquelle les particules (7) capables d'électroluminescence sont suspendues dans un milieu de réaction liquide se trouvant dans la chambre de réaction (5),
dans laquelle le milieu de réaction contient des photoréactifs (6), et
dans laquelle les particules (7) capables d'électroluminescence sont excitées au moyen d'une alimentation d'énergie à l'élément d'émission (4) pour émettre de la lumière activant les photoréactifs (6).

2. Utilisation selon la revendication 1, dans laquelle le milieu de réaction est aqueux et/ou organique.

3. Utilisation selon la revendication 1 ou 2,
dans laquelle les photoréactifs (6) comprennent des cellules ou organismes phototrophes vivants, en particulier microorganismes aquatiques, de préférence des micro-algues ou cyanobactéries, ou dans laquelle les photoréactifs (6) comprennent au moins une substance chimique, qui peut être excitée par l'absorption de lumière dans un état excité singulet ou triplet et qui libère l'énergie reçue par une réaction chimique avec d'autres réactifs dans le milieu de réaction.

4. Utilisation selon une des revendications 1 à 3, dans laquelle les particules (7) capables d'électroluminescence contiennent des substances électroluminophores et de préférence ont une structure âme-gaine, dans laquelle la substance électroluminophore se trouve dans l'âme, et la gaine consiste en un matériau transparent dans des conditions réactionnelles étant inerte dans le milieu de réaction.

5. Utilisation selon une des revendications 1 à 3, dans laquelle les particules (7) capables d'électroluminescence comprennent au moins un dispositif DEL (13) et au moins un élément d'antenne (10) électriquement raccordé au dispositif DEL (13) pour recevoir des champs électromagnétiques alternatifs irradiés et convertir les champs alternatifs reçus en énergie électrique.

6. Utilisation selon la revendication 5, dans laquelle les particules (7) comprennent une enveloppe (14) en matériau transparent dans des conditions réactionnelles étant inerte dans le milieu de réaction.

7. Utilisation selon une des revendications 1 à 6, dans laquelle l'élément d'émission (4) est réalisé de manière appropriée pour émettre des champs électromagnétiques alternatifs comme émetteur (4) inductif et/ou capacitif.

8. Utilisation selon une des revendications 1 à 7, dans laquelle les particules (7) sont formées à une densité moyenne, la différence entre celle-ci et la densité moyenne du milieu de réaction dans des conditions réactionnelles étant inférieure à 10 %, de préférence inférieure à 5 %, en particulier inférieure à 2 %.

9. Photoréacteur (1) comprenant une chambre de réaction (5), dans laquelle un milieu de réaction liquide est contenu,
dans lequel le milieu de réaction comporte des particules (7) capables d'électroluminescence, qui sont suspendues dans le milieu de réaction, et des photoréactifs (6),
dans lequel le réacteur (1) comprend au moins un élément d'émission (4) pour émettre des champs électromagnétiques alternatifs dans la chambre de réaction (5) du réacteur (1), qui est raccordé à une source d'énergie, et
dans lequel les particules (7) capables d'électroluminescence peuvent être excitées au moyen d'une alimentation d'énergie à l'élément d'émission (4) pour émettre de la lumière activant les photoréactifs (6).

10. Procédé pour cultiver des cellules ou organismes (6) phototrophes, en particulier des microorganismes aquatiques, de préférence des micro-algues ou cyanobactéries, dans lequel ces cellules ou organismes (6) sont cultivés, en particulier circulés, dans un milieu de réaction d'un photoréacteur (1) selon la revendication 9 sous alimentation d'énergie à l'élément d'émission (4) pendant une durée prédéterminée et dans des conditions réactionnelles prédéterminées.
